# EUROPÄISCHE PATENTANMELDUNG

(11) **EP 1 310 477 A2**
(43) Veröffentlichungstag der Anmeldung: **14.05.2003**
(21) Anmeldenummer: 02022735.1
(22) Anmeldetag: 11.10.2002
(51) Int. Cl.: C07C 51/377, C07C 319/20, C07C 51/08, C07C 253/08

(54) **Verfahren zur Herstellung von chiralen alpha-Hydroxycarbonsäuren**

(30) Priorität: 07.11.2001 AT 17482001
(71) Anmelder: DSM Fine Chemicals Austria Nfg GmbH & Co KG, 4021 Linz (AT)
(72) Erfinder: Mayrhofer, Herbert, 4210 Engerwitzdorf (AT); Neuhofer, Rudolf, 4210 Mittertreffling (AT); Poechlauer, Peter, 4040 Linz (AT); Skranc, Wolfgang, 1220 Wien (AT); Wirth, Irma, 4470 Enns (AT)
(74) Vertreter: Lindinger, Ingrid

(57) **Zusammenfassung**

Verfahren zur Herstellung von chiralen α-Hydroxycarbonsäuren der Formel 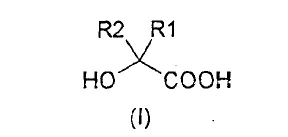 in der R1 einen gegebenenfalls durch Halogenatome substituierten C₁-C₂-Alkylrest und R2 einen gegebenenfalls durch Halogenatome substituierten C₂-C₃-Alkylrest bedeuten, aus einer Verbindung der Formel 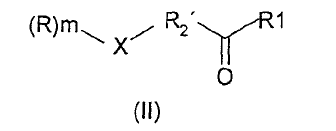 in der R1 wie oben definiert ist, R₂' einen gegebenenfalls durch Halogenatome substituierten C₂-C₃-Alkylenrest, m 0 oder 1 und R einen gegebenenfalls substituierten Alkylrest, einen Aryl-, Heteroaryl- oder einen Heterocyclusrest bedeuten und X Sauerstoff, Schwefel, Sulphinyl, Sulphonyl, Imino, C₁-C₆-Alkylimino, Xanthogenat, Silyl, oder, falls m gleich 0, Halogen sein kann,
die in Gegenwart eines Cyanidgruppendonors zu dem korrespondierenden (R)- oder (S)- Cyanhydrin oder dessen Racemat der Formel 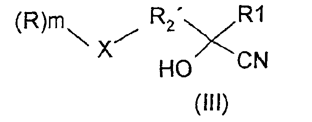 umgesetzt wird, dann durch saure Hydrolyse in eine Säure der Formel 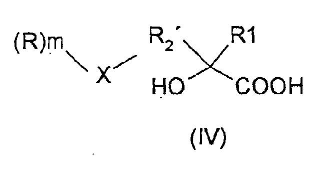 oder deren Racemat überführt wird, und durch Abspaltung der Gruppe der Formel

(R)m-X (V),

wobei gegebenenfalls zuerst eine Racematspaltung durchgeführt wird, die gewünschte chirale α-Hydroxycarbonsäure der Formel (I) erhalten wird.

## Beschreibung

Die Erfindung betrifft ein Verfahren zur Herstellung von chiralen α-Hydroxycarbonsäuren, die wertvolle Intermediate für pharmazeutische und agrochemische Produkte darstellen, aus C₄-C₆-Ketonen.

α-Hydroxycarbonsäuren, wie etwa 2-Hydroxy-2-methyl-3-phenylthio-propionsäure, werden beispielsweise gemäß EP 0 100 172 bei der Herstellung von Acylaniliden eingesetzt, die eine antiandrogene Aktivität besitzen. Gemäß EP 0 100 172 werden diese α-Hydroxycarbonsäuren als Racemat durch Umsetzung der entsprechenden Phenylthio-Ketone mit KCN zum korrespondierenden Cyanhydrin und anschließender Reaktion mit wässriger HCI erhalten. Ausbeuten, Reinheiten, sowie Angaben über die Konfiguration sind aus EP 0 100 172 nicht zu entnehmen.
Phenylthio-Ketone, die als Precursor für obige Reaktion dienen können, wie etwa 4-Phenylthio-2-butanon, werden beispielsweise gemäß J. Org. Chem. 1995, 60, 2022-2025, durch Addition von Thiophenol an das Methyl-Vinyl-Keton erhalten.
Gemäß J. Org. Chem. 1990, 55, 4643-4647, werden racemische tert. α-Benzyloxysäureester mittels Lipase OF von Candida cylindracea jeweils in den entsprechenden (+)- tert. α-Benzyloxysäureester und die korrespondierende (S)- α-Benzyloxycarbonsäure gespalten. Aus den (S)- α-Benzyloxycarbonsäuren werden sodann durch Hydrogenierung die gewünschten chiralen α-Hydroxycarbonsäuren, wie etwa (S)-2-Hydroxy-2-methylbuttersäure erhalten. Die Ausbeute liegt dabei bei 67%. Der ee-Wert des Endproduktes liegt lediglich bei 60%.

Aufgabe der vorliegenden Erfindung war es, ein Verfahren zur Herstellung von kurzkettigen C₄-C₆-α-Hydroxycarbonsäuren zu finden, das die Herstellung der gewünschten Produkte in hoher Ausbeute und Enantiomerenreinheit ermöglicht.

Unerwarteterweise konnte diese Aufgabe durch ein Verfahren gelöst werden, bei welchem kleinmolekulare, mit einer chemisch entfernbaren Gruppe derivatisierte C₄-C₆-Ketone durch Umsetzung mit einem Cyanidgruppendonor in Gegenwart einer Hydroxynitrillyase oder durch racemische Umsetzung, anschließender saurer Hydrolyse, gegebenenfalls Racematspaltung und Abspaltung der Gruppe in die entsprechende α-Hydroxycarbonsäuren überführt werden.

Gegenstand der Erfindung ist demnach ein Verfahren zur Herstellung von chiralen α-Hydroxycarbonsäuren der Formel (I) 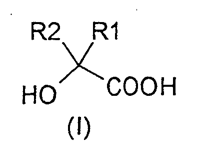 in der R1 einen gegebenenfalls durch ein- oder mehrere Halogenatome substituierten C₁-C₂-Alkylrest und R2 einen gegebenenfalls durch ein- oder mehrere Halogenatome substituierten C₂-C₃-Alkylrest bedeuten, das dadurch gekennzeichnet ist, dass eine Verbindung der Formel (II), 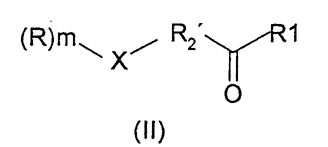 in der R1 wie oben definiert ist, R₂' einen gegebenenfalls durch ein- oder mehrere Halogenatome substituierten C₂-C₃-Alkylenrest bedeutet, m gleich 0 oder 1 sein kann, R einen C₁-C₂₀-Alkylrest, einen C₅-C₂₀-Aryl-, Heteroaryl- oder einen Heterocyclusrest bedeutet, wobei die Reste gegebenenfalls ein- oder mehrfach durch Substituenten aus der Gruppe C₁-C₄-Alkyl, C₁-C₄-Alkoxy, C₁-C₆-Alkylthio, Phenyl, Benzyl, Halogen, Hydroxy, Nitro, Carboxy, Ester, Thioester, Carbonate, Carbamate oder Urethane substituiert sein können, und X Sauerstoff, Schwefel, Sulphinyl, Sulphonyl, Imino, C₁-C₆-Alkylimino, Xanthogenat, Silyl, oder, falls m gleich 0, Halogen sein kann, in Gegenwart eines Cyanidgruppendonors entweder enantioselektiv mit einer (R)-oder (S)- Hydroxynitrillyase im organischen, wässrigen oder 2-Phasensystem oder in Emulsion zu dem korrespondierenden (R)- oder (S)-Cyanhydrin der Formel (III) 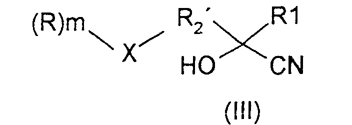 in der R1, R₂', R, m und X wie oben definiert sind, oder racemisch zum entsprechenden Racemat des Cyanhydrins der Formel (III) umgesetzt wird, sodann die Verbindung der Formel (III) oder dessen Racemat durch saure Hydrolyse in die entsprechende Säure der Formel (IV) 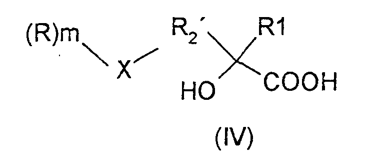 in der R1, R₂', R, m und X wie oben definiert sind, oder deren Racemat überführt wird, worauf die Abspaltung der Gruppe der Formel (V) 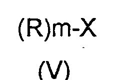 erfolgt, wobei im Falle des Racemates zuerst eine Racematspaltung durchgeführt wird, und die gewünschte chirale α-Hydroxycarbonsäuren der Formel (I) isoliert wird.

Bei dem erfindungsgemäßen Verfahren werden chirale α-Hydroxycarbonsäuren der Formel (I) 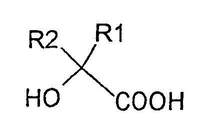 hergestellt.

In der Formel (I) bedeutet R1 einen gegebenenfalls durch ein- oder mehrere Halogenatome aus der Gruppe Fluor, Chlor, Brom, lod substituierten C₁-C₂-Alkylrest, wie etwa Methyl, Ethyl, Fluormethyl, Chlormethyl, Brommethyl, Difluormethyl, Dichlormethyl, Fluor-Chlor-methyl, Trifluormethyl, Trichlormethyl, Pentafluorethyl, Pentachlorethyl, u.s.w. Bevorzugt ist ein gegebenenfalls durch ein bis 3 Fluor- oder Chloratome substituierter Methylrest, besonders bevorzugt ist ein unsubstituierter Methylrest.
R2 bedeutet einen gegebenenfalls durch ein- oder mehrere Halogenatome aus der Gruppe Fluor, Chlor, Brom, Iod substituierten C₂-C₃-Alkylrest, wie etwa Ethyl, Difluorethyl, Propyl, Pentafluorethyl u.s.w. Bevorzugt bedeutet R2 einen gegebenenfalls durch ein- oder mehrere Fluor- oder Chloratome substituierten Ethylrest, besonders bevorzugt einen unsubstituierten Ethylrest.
Bevorzugt weist in den Verbindungen der Formel (I) R2 ein C-Atom mehr auf als R1. Beispiele für Verbindungen der Formel (I) sind (R)- oder (S)-2-Hydroxy-2-methylbutansäure, (R)- oder (S)-2-Hydroxy-2-ethyl-pentansäure, (R)- oder (S)-2-Hydroxy-2-fluormethyl-butansäure, (R)- oder (S)-2-Hydroxy-2-chlormethyl-butansäure, (R)- oder (S)-2-Hydroxy-2-difluormethyl-butansäure, (R)- oder (S)-2-Hydroxy-2-dichlormethylbutansäure (R)- oder (S)-2-Hydroxy-2-trifluormethyl-butansäure, (R)- oder (S)-3-Difluor-2-Hydroxy-2-methyl-butansäure, (R)- oder (S)-4-Difluor-2-hydroxy-2-methylbutansäure, (R)- oder (S)-3-Difluor-2-hydroxy-2-fluormethyl-butansäure, (R)- oder (S)-4-Difluor-2-hydroxy-2-fluormethyl-butansäure, (R)- oder (S)-3-Difluor-2-difluormethyl-2-hydroxy-butansäure, (R)- oder (S)-4-Difluor-2-difluormethyl-2-hydroxy-butansäure, (R)- oder (S)-3-Difluor-2-hydroxy-2-trifluormethyl-butansäure, (R)- oder (S)-3-Difluor-2-hydroxy-2-trifluormethyl-butansäure, (R)- oder (S)-4-Difluor-2-hydroxy-2-trifluormethyl-butansäure, (2S,3S)-3-Fluor-2-Hydroxy-2-methyl-butansäure, (2S,3R)-3-Fluor-2-Hydroxy-2-methyl-butansäure, (2R,3S)-3-Fluor-2-Hydroxy-2-methyl-butansäure, (2R,3R)-3-Fluor-2-Hydroxy-2-methyl-butansäure, u.s.w.

Als Ausgangsprodukt dient die Verbindung der Formel (II)

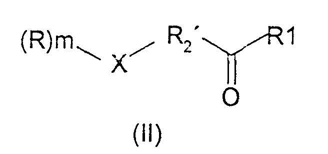

In der Formel (II) ist R1 wie oben definiert.

R₂' bedeutet einen gegebenenfalls durch ein- oder mehrere Halogenatome aus der Gruppe Fluor, Chlor, Brom lod substituierten C₂-C₃-Alkylenrest, wie etwa Ethylen, Propylen, Difluorethylen, Pentafluorethylen, Dichlorethylen, Pentachlorethylen u.s.w. Bevorzugt bedeutet R₂' einen gegebenenfalls durch ein- oder mehrere Fluor- oder Chloratome substituierten Ethylenrest, besonders bevorzugt einen unsubstituierten Ethylenrest.

m kann gleich 0 oder 1 sein und R einen C₁-C₂₀-Alkylrest, einen C₅-C₂₀-Aryl-, Heteroaryl- oder einen Heterocyclusrest bedeuten.

Unter Alkyl sind dabei gesättigte oder ein- oder mehrfach ungesättigte, lineare, verzweigte oder cyclische, primäre, sekundäre oder tertiäre Hydrocarbonreste zu verstehen. Dies sind C₁-C₂₀-Alkylreste, wie etwa Methyl, Ethyl, Propyl, iso-Propyl, Butyl, iso-Butyl, t-Butyl, Pentyl, Cyclopentyl, iso-Pentyl, neo-Pentyl, Hexyl, iso-Hexyl, Cyclohexyl, Cyclohexylmethyl, 3-Methylpentyl, 2,2-Dimethylbutyl, 2,3-Dimethylbutyl, Octyl, Cyclooctyl, Decyl, Cyclodecyl, Dodecyl, Cyclododecyl u.s.w.
Bevorzugt sind dabei C₁-C₁₂-Alkylreste und besonders bevorzugt C₂-C₈-Alkylreste. Die Alkylgruppe kann gegebenenfalls ein- oder mehrfach durch unter den Reaktionsbedingungen inerte Substituenten aus der Gruppe C₁-C₄-Alkoxy, C₁-C₆-Alkylthio, Phenyl, Benzyl, Halogen, Hydroxy, Nitro, Carboxy Ester, Thioester, Carbonate, Carbamate oder Urethane substituiert sein.

Unter Aryl sind bevorzugt C₆-C₂₀-Arylgruppen zu verstehen, wie etwa Phenyl, Biphenyl, Naphthyl, Indenyl, Fluorenyl u.s.w.

Die Arylgruppe kann dabei gegebenenfalls durch unter den Reaktionsbedingungen inerte Substituenten aus der Gruppe C₁-C₄-Alkyl, C₁-C₄-Alkoxy, C₁-C₆-Alkylthio, Phenyl, Benzyl, Halogen, Hydroxy, Nitro, Carboxy Ester, Thioester, Carbonate, Carbamate oder Urethane ein- oder mehrfach substituiert sein.

Unter Heteroaryl oder Heterocyclus sind cyclische Reste zu verstehen die zumindestens ein S, O- oder N-Atom im Ring enthalten. Dies sind beispielsweise Furyl, Thienyl, Pyridyl, Pyrimidyl, Imidazolyl, Thiazol, Tetrazolyl, Pyrazinyl, Benzofuranyl, Benzthiazol, Chinolyl, Isochinolyl, Isobenzofuryl, Pyrazolyl, Indolyl, Isoindolyl, Benzoimidazolyl, Purinyl, Carbazolyl, Oxazolyl, Isoxazolyl, Pyrrolyl, Chinazolinyl, Pyridazinyl, Phthalazinyl u.s.w.

Funktionelle O- oder N-Gruppen können dabei nötigenfalls geschützt werden.
Die Heteroarylgruppe bzw. der Heterocyclus kann dabei gegebenenfalls ein- oder mehrfach durch die bereits oben angeführten Substituenten substituiert sein.

Bevorzugt bedeutet m gleich 1 und R einen C₅-C₂₀-Aryl- oder Heteroarylrest, wie Phenyl, Naphthyl, Pyridyl, Pyrimidinyl, Benzthiazol, die gegebenenfalls ein- oder zweifach durch Methyl, Fluor, Chlor, Hydroxy oder Nitro substituiert sein können.
Besonders bevorzugt bedeutet R Phenyl.

X kann in der Verbindung der Formel (II) Bor, Sauerstoff, Schwefel, Sulphinyl (-SO-), Sulphonyl (-SO₂-), Imino (-NH-), C₁-C₆-Alkylimino, Xanthogenat (-O-CS-S-), Silyl (-Si-), oder, falls m gleich 0, Halogen sein.
Bevorzugt bedeutet X Schwefel, Sulphinyl (-SO-), Sulphonyl (-SO₂-), Xanthogenat (-O-CS-S-) oder Silyl (-Si-).
Geeignete Ausgangsverbindungen sind beispielsweise 4-Phenylthio-2-butanon, 4-Phenylthio-4-difluor-2-butanon, 4-Phenylthio-3-difluor-2-butanon, 4-Phenylthio-1-fluor-2-butanon, 4-Phenylthio-1-difluor-2-butanon, 4-Phenylthio-1-trifluor-2-butanon, 4-Trimethylsilyl-2-butanon, 4-Dimethyphenyllsilyl-2-butanon, 4-Diphenylmethylsilyl-2-butanon, 4-Triphenylsilylbutanon, 4-(9-Borabicyclo[3.3.1]nonan-9-yl)-2-butanon, 4-Diphenylboran-2-butanon, 4-Benzylimino-2-butanon, 4-Phenylsulphinyl-2-butanon, 4-Phenylsulphonyl-2-butanon, O-(1-Methylethyl)-S-(3-oxybutyl)-dithiocarbonat, O-(Phenyl)-S-(3-oxybutyl)-dithiocarbonat, u.s.w..

Die Verbindungen der Formel (II) können beispielsweise analog J. Org. Chem. 1995, 60, 2022-2025, Khim. Khim. Tekhnol. (1980), 23(7), 836f. oder analog Zhur. org. Khim., 1971, 7, 2221 aus den entsprechenden Ketonen hergestellt werden. Verbindungen der Formel (II), in denen X Sulphinyl oder Sulphonyl bedeutet, können beispielsweise auch durch Oxidation der korrespondierenden Verbindung der Formel (I), in der X Schwefel bedeutet, hergestellt werden.
Bevorzugt werden die Verbindungen der Formel (II) gemäß einer leicht modifizierten Variante aus J. Org. Chem. 1995, 60, 2022-2025 hergestellt.
So wird beispielsweise 4-Phenylthio-2-butanon durch Reaktion von Thiophenol mit Methylvinylketon in Gegenwart eines tert. Amins, beispielsweise Triethylamin, als Katalysator in einem geeigneten Lösungsmittel, wie etwa Methyl - tert. butylether (MTBE) bei 0°C-10°C umgesetzt und 4-Phenylthio-2-butanon durch Extraktion isoliert.
Wird im nächsten Schritt, der Umsetzung mit einem Cyanidgruppendonor, das gleiche Lösungsmittel, beispielsweise MTBE, eingesetzt, so muss die Verbindung der Formel (II) nicht vollständig isoliert werden, es kann die organische Phase, die die Verbindung der Formel (II) enthält, direkt eingesetzt werden. Wird bei diesen beiden Schritten ein unterschiedliches Lösungsmittel eingesetzt, so kann es notwendig sein, einen Lösungsmitteltausch durchzuführen.
Bevorzugt wird bei der Herstellung der Verbindung der Formel (II) das gleiche Lösungsmittel, wie in der nachfolgenden Umsetzung mit dem Cyanidgruppendonor verwendet.

Die Verbindung der Formel (II) wird sodann mit einem Cyanidgruppendonor umgesetzt, wobei die Umsetzung enantioselektiv in Anwesenheit einer (R)- oder (S)-Hydroxynitrillyase (HNL) oder auf racemischen, basenkatalysiertem Weg, beispielsweise unter Verwendung von Amberlyst, Triethylamin, Diazabicyclooctan, Pyridin oder einer anderen organischen Base, erfolgen kann.
Als Cyanidgruppendonor kommen Blausäure, Alkali-Cyanide oder ein Cyanhydrin der allgemeinen Formel (VI)

R₃R₄C(OH)(CN),

in Betracht. In der Formel (VI) bedeuten R₃ und R₄ unabhängig voneinander Wasserstoff oder eine unsubstituierte Kohlenwasserstoffgruppe, oder R₃ und R₄ gemeinsam eine Alkylengruppe mit 4 oder 5 C-Atomen, wobei R₃ und R₄ nicht gleichzeitig Wasserstoff bedeuten. Die Kohlenwasserstoffgruppen sind aliphatische oder aromatische, bevorzugt aliphatische Gruppen. Bevorzugt bedeuten R₃ und R₄ Alkylgruppen mit 1 - 6 C-Atomen, ganz bevorzugt ist Acetocyanhydrin als Cyanidgruppendonor der Formel (VI).
Die Herstellung des Cyanidgruppendonors kann nach bekannten Verfahren erfolgen. Cyanhydrine, insbesondere Acetocyanhydrin, sind auch käuflich zu erwerben. Bevorzugt wird Blausäure (HCN), KCN, NaCN, oder Acetocyanhydrin, besonders bevorzugt Blausäure als Cyanidgruppendonor eingesetzt.

Die Blausäure kann dabei auch erst kurz vor der Reaktion aus einem ihrer Salze wie etwa NaCN oder KCN freigesetzt und in Substanz oder in gelöster Form dem Reaktionsgemisch zugegeben werden.

Der Cyanidgruppendonor wird dabei in einem molaren Verhältnis zur Verbindung der Formel (II) von 0,5:1 bis 5:1, bevorzugt von 0,8:1 bis 4:1 und besonders bevorzugt von 1:1 bis 3:1, eingesetzt.
Die Umsetzung kann im organischen, wässrigen oder 2-Phasensystem oder in Emulsion durchgeführt werden.

Als organisches Verdünnungsmittel können mit Wasser nicht oder geringfügig mischbare aliphatische oder aromatische Kohlenwasserstoffe, die gegebenenfalls halogeniert sind, Alkohole, Ether oder Ester oder Gemische davon verwendet werden. Bevorzugt werden Methyl -tert. Butylether (MTBE), Diisopropylether, Dibutylether und Ethylacetat oder deren Gemisch eingesetzt.
Bei der enantioselektiven Umsetzung wird als wässriges System eine wässrige, die entsprechende HNL enthaltende Lösung oder Pufferlösung verwendet. Beispiele dafür sind Na-Citratpuffer, Phosphatpuffer u.s.w.
Die HNLs können dabei entweder als solche oder immobilisiert in dem organischen Verdünnungsmittel vorliegen, die Umsetzung kann jedoch auch in einem Zweiphasensystem oder in Emulsion mit nicht-immobilisierter HNL erfolgen.
Als HNLs eignen sich sowohl native als auch rekombinante (R)- und (S)-HNLs, wie sie aus dem Stand der Technik, beispielsweise aus EP 0 969 095, EP 0 951 561, EP 0 927 766, EP 0 632 130, EP 0547 655, EP 0 326 063, WO 01/44487 u.s.w. bekannt sind.
Die basen- oder enzymkatalysierte Addition der Cyanidgruppe an die entsprechende Verbindung der Formel (II) kann dabei analog dem Stand der Technik, beispielsweise analog EP 0 969 095, EP 0 951 561, EP 0 927 766, EP 0 632 130, EP 0547 655, EP 0 326 063 u.s.w., erfolgen.

Das entsprechende *(R)-* und *(S)-* Cyanhydrin der Formel (III) oder dessen Racemat kann sodann, beispielsweise nach Extraktion oder gegebenenfalls nach Abfiltrieren des Enzyms und Abdestillieren des Lösungsmittels, ohne weitere Reinigung analog dem Stand der Technik, beispielsweise wie in Angew. Chem. 1994, 106, S 1615 oder in Tetrahedron Letters, Vol. 31, No. 9, pp1249-1252, 1990 beschrieben, mit konzentrierter Salzsäure hydrolysiert werden. Für die Hydrolyse können auch andere geeignete Säuren, wie etwa H₂SO₄ verwendet werden, bevorzugt wird jedoch HCI eingesetzt.

Die Aufarbeitung der Reaktionslösung, die die so erhaltenen rohen (*R*)- oder (*S*)-α-Hydroxycarbonsäuren, die in etwa die gleiche optische Reinheit wie die korrespondierenden *(R)*- und *(S)*- Cyanhydrine aufweisen, enthält, kann sodann entweder direkt oder nach Verdünnen der erhaltenen Reaktionslösung mit Wasser und anschließender Extraktion mit MTBE, Toluol, Xylol, (Poly)ethern, halogenierten Kohlenwasserstoffen, Acetonitril u.s.w. zwischen 10-99°C, bevorzugt zwischen 15-95°C und besonders bevorzugt zwischen 20-90°C erfolgen. Bevorzugt werden dabei Toluol oder Xylol verwendet.
Zur Erreichung einer Anreicherung des gewünschten Enantiomerens wird die Extraktionslösung bevorzugt eingeengt und stark auf bis zu -10°C abgekühlt. Daraufhin werden die entstandenen Kristalle abgetrennt, beispielsweise über eine Nutsche, bevorzugt in einem aromatischen Kohlenwasserstoff, gegebenenfalls in Anwesenheit eines Cosolvens, umkristallisiert und das Produkt mit einem höheren ee als bei der Cyanhydrinstufe erhalten.

Weiters ist es möglich, den Kristallisationsschritt direkt an den Hydrolyseschritt zu koppeln, sodass das Extrahieren der Hydroxycarbonsäure mittels Ethern entfällt. Dazu wird der Hydrolyselösung bei Hydrolysetemperatur ein aromatischer Kohlenwasserstoff, gegebenenfalls in Kombination mit einem Cosolvens, zugesetzt und das Reaktionsgemisch extrahiert. Die wässrigen Phasen werden verworfen, worauf die vereinigten organischen Phasen bevorzugt nach Einengen abgekühlt werden und die hochreinen Hydroxycarbonsäuren auskristallisieren.

Wird das Racemat der α-Hydroxycarbonsäure (IV) erhalten, so erfolgt zuerst die Racemattrennung mit einer Spaltbase. Als Spaltbase eignen sich beispielsweise chirale Amine, etwa solche, wie sie beispielsweise in T. Vries et al., Angew Chem., Int. Ed., (1998), 37, S.2349-2354 beschrieben sind, wie etwa (R)- oder (S)- Phenylethylamin, (L)- oder (D)- Phenylglycinamid, (L)- oder (D)-Norephedrin, (R)- oder (S)- Naphtylethylamin u.s.w..

Die Spaltung erfolgt in einem Lösungsmittel, in dem sich die α-Hydroxycarbonsäure löst, beispielsweise in Isopropylacetat (IPA), Ethylacetat, Toluol, Xylol. Nach der Zugabe der Spaltbase wird unter Rühren mit der Kristallisation durch Abkühlen des Reaktionsgemisches begonnen. Bei der Kristallisation ist es auch möglich diese durch Animpfen zu unterstützen bzw. auszulösen. Die Abkühlung erfolgt auf bis zu -15°C, bevorzugt auf bis zu etwa +10°C.
Nach beendeter Kristallisation wird filtriert, die Kristalle gegebenenfalls gewaschen und anschließend gegebenenfalls getrocknet.
Zur Freisetzung der entsprechenden chiralen Säure wird sodann zu einem Gemisch aus chiralem Salz, Wasser und einem geeigneten Lösungsmittel HCl zugegeben, wodurch zwei klare Phasen erhalten werden. Als Lösungsmittel eignen sich solche, die mit Wasser nicht mischbar sind, die die Säure lösen und die gegen HCl resistent sind. Beispiele dafür sind Ether oder Kohlenwasserstoffe. Bevorzugt werden Ether eingesetzt, besonders bevorzugt MTBE.

Liegt sodann die entsprechende enantiomerenangereicherte α-Hydroxycarbonsäure in gereinigter Form vor, erfolgt die Abspaltung der chemisch entfernbaren Gruppe der Formel (V).
Dies erfolgt beispielsweise bei X=S und Derivaten davon mittels Raney Ni, NiCl₂ und NaBH4, Cl₂, wobei entstehende Halogenide z.B. durch Zink entfernt werden müssen, wie etwa analog J. Org. Chem., Vol. 58, No 9, 1993, 2407-2413.
Ist X=O, so kann die Gruppe RmO durch Eliminierung abgespaltet werden, wobei in diesem Fall durch Eliminierung eine ungesättigte α-Hydroxycarbonsäure entsteht, die gegebenenfalls hydriert werden muss.
Ist X=Si, so kann die Gruppe RmSi durch basische Aufarbeitung (K₂CO₃ in Methanol), durch Behandlung mit Fluoridionen (KF, NaF) oder konz HCI für mehrere Stunden entfernt werden.
Durch das erfindungsgemäße Verfahren, werden chirale α-Hydroxycarbonsäuren aus C₄-C₆-Ketone in hohen Ausbeuten und in hoher Enantiomerenreinheit erhalten, wobei die enantioselektive HCN-Addition wesentlich selektiver als gemäß dem Stand der Technik abläuft und die Racematspaltung nach der racemischen HCN-Addition mit einfacheren Trennreagenzien als gemäß dem Stand der Technik notwendig durchgeführt werden kann.

### Beispiel 1: Herstellung der Verbindung der Formel (II) (4-Phenyithio-2-butanon) analog J. Org. Chem. 1995, 60, 2022-2025

113,5 g (1,03 mol) Thiophenol und 1,0 g (9,8 mmol) Triethylamin wurden bei 0°C in 250 ml MTBE eingebracht. Zu dieser kalten Lösung wurden innerhalb einer Stunde 70,1 g (1,03 mol) Methylvinylketon zugetropft, sodass die Temperatur zwischen 0-10°C gehalten wurde. Anschließend wurde das Reaktionsgemisch bei 10°C für 1 Stunde nachgerührt. Die Reaktionslösung wurde mit weiteren 250 ml MTBE versetzt und mit 200 ml 4 % NaOH Lösung extrahiert. Die MTBE-Phase wurde mit 200 ml konz. NaCI Lösung extrahiert und anschließend zur Trockene eingeengt.
Ausbeute: 164,6 g 4-Phenylthio-2-butanon (91,3 % d. Th.)

### Beispiel 2: Herstellung der Verbindung der Formel (III) ((S)-2-Hydroxy-2-methyl-4-phenyl-thiobuttersäurenitril)

11,35 g Thiophenol und 0,1 g Triethylamin wurden bei 0°C in 25 ml MTBE eingebracht. Zu dieser kalten Lösung wurden innerhalb einer Stunde 7,01 g Methylvinylketon zugetropft, sodass die Temperatur zwischen 0-10°C gehalten wurde. Anschließend wurde die Reaktionslösung bei 10°C für 2 Stunden nachgerührt. Die Reaktionslösung wurde sodann mit weiteren 25 ml MTBE versetzt und mit 25 ml 4 % NaOH Lösung extrahiert. Die organische Phase wurde mit 25 ml dest. Wasser extrahiert und anschließend mit 50 ml Pufferlösung (K₂HPO₄/Citrat 30 mmol, pH 6,0) versetzt und 10 min gerührt. Der pH der zweiphasigen Mischung wurde mit 10 %iger wässriger Citronensäure auf pH 6,00 gestellt.
Zu dieser zweiphasigen Mischung wurden 4,5 ml S-HNL von Hevea brasiliensis (6930 IU/mL) hinzugegeben und dieses Reaktionsmischung bei 0°C zur Emulsion geschlagen. Daraufhin wurden innerhalb von 1 Stunde bei 0°C 8,0 ml HCN hinzugegeben. Nach weiteren 3 Stunden Rühren, wurde der Reaktionslösung 50 ml MTBE zugemischt, 10 min gerührt und die Phasen getrennt. Die wässrige Phase wurde neuerlich mit 25 ml MTBE extrahiert und die vereinten organischen Phasen am Rotavapor vom Lösungsmittel befreit.
Ausbeute: 17,3 g 2-Hydroxy-2-methyl-4-phenylthiobuttersäurenitril (88,13 % d.T.), 94,09 % ee

Der ee-Wert wurde auf einer CP-Chirasil-Dex-CB Säule bestimmt, wobei das Cyanhydrin mit 1-Trimethylsilyl-imidazol in Dichlormethan derivatisiert wurde.

### Beispiel 3: Herstellung der Verbindung (S)-2-Hydroxy-2-methyl-4-phenylthiobuttersäure

100,0 g (0,509 mol) (S)-2-Hydroxy-2-methyl-4-phenyl-thiobuttersäurenitril (ee: 95,45 %) wurden zu 800 ml konz HCl bei 65°C hinzugegeben und 90 min lang gerührt. Daraufhin wurde die Temperatur auf 85°C gesteigert und bei dieser Temperatur 120 min reagieren gelassen. Daraufhin wurde bei 85°C 200 ml Toluol zugesetzt und 15 min bei 50°C gerührt. Nach der Phasenseparation wurde die organische Phase abgezogen und die wässrige Phase neuerlich mit 50 ml Toluol extrahiert. Die vereinten organischen Phasen wurden unter Zusatz von 100 ml Toluol (zwecks der leichteren Filtrierbarkeit) auf 0°C abgekühlt. Die ausgefallenen Kristalle wurden abfiltriert und getrocknet.
Ausbeute: 55,2 g (S)-2-Hydroxy-2-methyl-4-phenyl-thiobuttersäure (47,9 % d. Th.), > 99,0% ee

### Beispiel 4: Synthese von racemischen (R/S)-2-Hydroxy-4-phenylthio-butyronitril

9,01 g (50 mmol) 4-Phenylthio-2-butanon wurden in 30 ml MTBE gelöst und mit 1,0 g lonentauscher Amberlyst A-21 versetzt. Unter Rühren wurde daraufhin 9,8 ml (250 mmol) HCN hinzugegeben und 2 Stunden bei Raumtemperatur gerührt. Der lonentauscher wurde abfiltriert und zur Lösung 0,05 g Zitronensäure hinzugegeben. Am Rotationsverdampfer wurde das Lösungmittel und HCN bei 40°C unter Vakuum abgezogen. Es wurden 9,5 g von (R/S)-2-Hydroxy-4-phenylthio-butyronitril ( 91,7 % d. Th.) gefunden.

### Beispiel 5: Synthese von rac-2-Hydroxy-2-methyl-4-phenylthio-buttersäure

7,75 g (39,5mmol) rac-2-Hydroxy-2-methyl-4-phenythiobuttersäurenitril, hergestellt nach Beispiel 4, wurden mit 77,5 g konz. HCI versetzt und bei 65°C bis zum Verschwinden des Cyanhydrins gerührt. Anschließend wurde die Temperatur auf 85°C erwärmt und 6 Stunden gerührt. Der Ansatz wurde mit 100 mL Wasser versetzt, auf Raumtemperatur abgekühlt und dreimal mit MTBE extrahiert. Die vereinigten organischen Phasen wurden über Na2SO4 getrocknet und zur Trockenen eingedampft. Das Rohprodukt wurde mit Heptan digeriert.
Ausbeute: 6,80 g rac-2-Hydroxy-2-methyl-4-phenyithio-buttersäure (80,3 % d. Th.)

### Beispiel 6: Racematspaltung:

5,00 g (22,1 mmol) rac. 2-Hydroxy-2-methyl-4-phenylthiobuttersäure, hergestellt nach Beispiel 5 wurde in Ethylacetat gelöst (60°C) und mit 2,67 g (22,1 mmol) S-(-)-Phenylethylamin versetzt. Anschließend wurde ein drittel Volumen von Ethylacetat an DIPE zugesetzt. Beim Abkühlen wurde mit Impfkristallen angeimpft, sodass nach dem Abkühlen auf 10°C das Diastereomeren Salz aus S-Phenylethylamin und R-2-Hydroxy-2-methyl-4-phenylthiobuttersäure auskristallisierte. Das Salz wurde abgesaugt, in Salzsäure aufgekocht und mit MTBE extrahiert. Die organische Phase wurde abgezogen und R-2-Hydroxy-2-methyl-4-phenylthiobuttersäure in einem R/S Verhältnis von über 3:1 und einer Ausbeute von 80 % erhalten.

### Beispiel 7: Synthese von (S)-2-Hydroxy-2-methylbuttersäure

25,0 g (0,110) (S)-2-Hydroxy-2-methyl-4-phenylthiobuttersäure (99,4 % ee), hergestellt analog Beispiel 3, wurden in 55,2 mL 2 M Natronlauge gelöst und unter Argonatmosphäre auf 70-74°C erwärmt. Anschließend wurde innerhalb von 2 Stunden 190,0 g Raney Ni portionsweise zugegeben und bei 70-74°C 3 Stunden nachgerührt. Der Katalysator wurde abfiltriert und mit Wasser gewaschen. Das Filtrat wurde einmal mit MTBE extrahiert. Die wässrige Phase (pH 9,8) wurde mit konz. HCI auf pH 1 angesäuert und dreimal mit MTBE extrahiert, wobei mit NaCI ausgesalzt wurde. Die vereinten organischen Phasen wurden mit Natriumsulfat getrocknet und zur Trockene eingedampft.
12,21 g Rohprodukt wurden aus 400 mL n-Hexan umkristallisiert.
Ausbeute: 11,6 g (S)-2-Hydroxy-2-methylbuttersäure (99,4 % ee) (88,9 % d. Th.)

### Beispiel 8: Synthese von (S)-2-Hydroxy-2-methylbuttersäure

5,0 g (2,20mmol) (S)-2-Hydroxy-2-methyl-4-phenylthiobuttersäure (99,0 % ee), hergestellt nach Beispiel 3, wurden in 44 ml Methanol/Tetrahydrofuran (3:1) gelöst und mit 23,6 g NiCl₂ versetzt. Die grüne Lösung wurde auf 3°C abgekühlt und innerhalb von 180 min zwischen 10-25°C mit 11,3 g Natriumborhydrid versetzt und über Nacht gerührt. Anschließend wurden 2,6 g NiCl₂ in 20 ml Methanol/Tetrahydrofuran (3:1) zugegeben und innerhalb von 30 min 1,3 g NaBH4 bei maximal 20°C eingetragen. Die Lösung wurde eingedampft wobei 30 mL Wasser zugegeben wurde. Nach Zugabe von 30 ml halb konzentrierter HCI und 30 ml MTBE wurde nach einiger Zeit der Niederschlag abfiltriert, das zweiphasige Gemisch mit NaCI versetzt und die Phasen getrennt. Die wässrige Phase wurde noch zweimal mit MTBE extrahiert. Die vereinten organischen Phasen wurden mit Natriumsulfat getrocknet und zur Trockene eingedampft.
Ausbeute: 2,33 g (S)-2-Hydroxy-2-methylbuttersäure (97,6 % ee) (89,2 % d. Th.)

## Patentansprüche

1. Verfahren zur Herstellung von chiralen α-Hydroxycarbonsäuren der Formel (I) 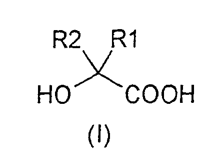 in der R1 einen gegebenenfalls durch ein- oder mehrere Halogenatome substituierten C₁-C₂-Alkylrest und R2 einen gegebenenfalls durch ein- oder mehrere Halogenatome substituierten C₂-C₃-Alkylrest bedeuten, **dadurch gekennzeichnet, dass** eine Verbindung der Formel (II) 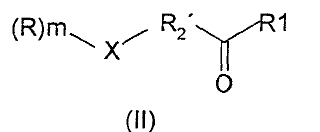 in der R1 wie oben definiert ist, R₂' einen gegebenenfalls durch ein- oder mehrere Halogenatome substituierten C₂-C₃-Alkylenrest bedeutet, m gleich 0 oder 1 sein kann, R einen C₁-C₂₀-Alkylrest, einen C₅-C₂₀-Aryl-, Heteroaryl- oder einen Heterocyclusrest bedeutet, wobei die Reste gegebenenfalls ein- oder mehrfach durch Substituenten aus der Gruppe C₁-C₄-Alkyl, C₁-C₄-Alkoxy, C₁-C₆-Alkylthio, Phenyl, Benzyl, Halogen, Hydroxy, Nitro, Carboxy, Ester, Thioester, Carbonate, Carbamate oder Urethane substituiert sein können, und X Sauerstoff, Schwefel, Sulphinyl, Sulphonyl, Imino, C₁-C₆-Alkylimino, Xanthogenat, Silyl, oder, falls m gleich 0, Halogen sein kann,
in Gegenwart eines Cyanidgruppendonors entweder enantioselektiv mit einer (R)-oder (S)-Hydroxynitrillyase im organischen, wässrigen oder 2-Phasensystem oder in Emulsion zu dem korrespondierenden (R)- oder (S)-Cyanhydrin der Formel (III) 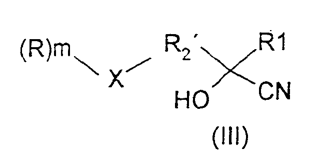 in der R1, R₂', R, m und X wie oben definiert sind, oder racemisch zum entsprechenden Racemat der Cyanhydrins der Formel (III) umgesetzt wird, sodann die Verbindung der Formel (III) oder dessen Racemat durch saure Hydrolyse in die entsprechende Säure der Formel (IV) 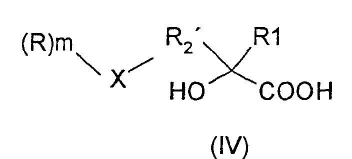 in der R1, R₂', R, m und X wie oben definiert sind, oder deren Racemat überführt wird, worauf die Abspaltung der Gruppe der Formel (V)
(R)m-X
erfolgt, wobei im Falle des Racemates zuerst eine Racematspaltung durchgeführt wird, und die gewünschte chirale α-Hydroxycarbonsäuren der Formel (I) isoliert wird.

2. Verfahren nach Anspruch 1, **dadurch gekennzeichnet, dass** in der Formel (I) R1 einen gegebenenfalls durch ein bis drei Fluor- oder Chloratome substituierten Methylrest und R2 einen gegebenenfalls durch ein bis drei Fluor- oder Chloratome substituierten Ethylrest bedeuten und in der Formel (II) R1 einen gegebenenfalls durch ein bis drei Fluor- oder Chloratome substituierten Methylrest, R₂' einen gegebenenfalls durch ein bis drei Fluor- oder Chloratome substituierten Ethylenrest bedeuten, m gleich 1 ist, R einen C₅-C₂₀-Aryl- oder Heteroarylrest sein kann, der gegebenenfalls ein- oder zweifach durch Methyl, Fluor, Chlor, Hydroxy oder Nitro substituiert sein kann und X Schwefel, Sulphinyl, Sulphonyl, Xanthogenat, oder Silyl bedeutet.

3. Verfahren nach Anspruch 1, **dadurch gekennzeichnet, dass** als Cyanidgruppendonor Blausäure, Alkali-Cyanide oder ein Cyanhydrin der allgemeinen Formel (VI)
R₃R₄C(OH)(CN),
in der R₃ und R₄ unabhängig voneinander Wasserstoff oder eine unsubstituierte aliphatische oder aromatische Kohlenwasserstoffgruppe sein können, oder R₃ und R₄ gemeinsam eine Alkylengruppe mit 4 oder 5 C-Atomen, wobei R₃ und R₄ nicht gleichzeitig Wasserstoff bedeuten.

4. Verfahren nach Anspruch 1, **dadurch gekennzeichnet, dass** als organisches Lösungsmittel mit Wasser nicht oder geringfügig mischbare aliphatische oder aromatische Kohlenwasserstoffe, die gegebenenfalls halogeniert sind, Alkohole, Ether oder Ester oder Gemische davon verwendet werden, oder dass bei der enantioselektiven Umsetzung als wässriges System eine wässrige, die entsprechende Hydroxynitrillyase enthaltende Lösung oder Pufferlösung verwendet wird.

5. Verfahren nach Anspruch 1, **dadurch gekennzeichnet, dass** bei der enantioselektiven Umsetzung sowohl native als auch rekombinante (R)- und (S)-Hydroxynitrillyasen eingesetzt werden können.

6. Verfahren nach Anspruch 1, **dadurch gekennzeichnet, dass** das (R )- oder (S)-Cyanhydrin der Formel (III) mit HCl oder H₂SO₄ hydrolysiert wird, die Reaktionslösung gegebenenfalls nach Verdünnen mit Wasser mit einem geeigneten Extraktionsmittel aus der Gruppe Methyl-tert.-butylether, Toluol, Xylol, (Poly)ethern, halogenierten Kohlenwasserstoffen oder Acetonitril bei 10 bis 99°C extrahiert wird, die Extraktionslösung auf bis zu -10°C abgekühlt wird und die entstandenen Kristalle der entsprechenden (R )- oder (S)-α-Hydroxycarbonsäuren der Formel (IV) abgetrennt und gegebenenfalls in einem aromatischen Kohlenwasserstoff, gegebenenfalls in Anwesenheit eines Cosolvens umkristallisiert werden.

7. Verfahren nach Anspruch 1, **dadurch gekennzeichnet, dass** racemisches Cyanhydrin der Formel (III) mit HCI oder H₂SO₄ hydrolysiert wird, die Reaktionslösung gegebenenfalls nach Verdünnen mit Wasser mit einem geeigneten Extraktionsmittel aus der Gruppe Methyl-tert.-butylether, Toluol, Xylol, (Poly)ethern, halogenierten Kohlenwasserstoffen oder Acetonitril bei 10 bis 99°C extrahiert wird, worauf das Racemat der α-Hydroxycarbonsäuren der Formel (IV) mit einem chiralen Amin als Spaltbase in einem Lösungsmittel, in dem sich die Hydroxycarbonsäure löst, gespalten und das entsprechende chirale Salz auskristallisiert wird, das zur Freisetzung der entsprechenden chiralen Säure in Wasser und einem geeigneten Lösungsmittel mit HCI versetzt wird.

8. Verfahren nach Anspruch 1, **dadurch gekennzeichnet, dass** die Abspaltung der Gruppe der Formel (V) bei X gleich Schwefel und dessen Derivaten mittels Raney Ni, NiCl₂ und NaBH₄, Chlor erfolgt, wobei entstehende Halogenide entfernt werden müssen, oder bei X gleich Sauerstoff durch Eliminierung, wodurch eine ungesättigte α-Hydroxycarbonsäure erhalten wird, die gegebenenfalls hydriert werden kann, oder bei X gleich Si durch basische Aufarbeitung, durch Behandlung mit Fluoridionen oder mittels HCI erfolgt.
